# EUROPÄISCHE PATENTSCHRIFT

(11) **EP 3 659 561 B1**
(45) Veröffentlichungstag und Bekanntmachung des Hinweises auf die Patenterteilung: **30.03.2022**
(21) Anmeldenummer: 19211486.6
(22) Anmeldetag: 26.11.2019
(51) Int. Cl.: A61F 5/02

(54) **RÜCKENORTHESE ZUM STABILISIEREN DES RÜCKENS EINES PATIENTEN UND VERFAHREN ZUM ANLEGEN DER RÜCKENORTHESE**
BACK ORTHOSIS FOR STABILIZING THE BACK OF A PATIENT AND METHOD OF APPLYING THE BACK ORTHOSIS
ORTHÈSE VERTÉBRALE POUR STABILISER LE DOS D'UN PATIENT ET MÉTHODE D'APPLICATION DE L'ORTHÈSE VERTÉBRALE

(30) Priorität: 27.11.2018 DE 102018009316
(43) Veröffentlichungstag der Anmeldung: 03.06.2020
(73) Patentinhaber: Bort GmbH, 71384 Weinstadt-Benzach (DE)
(72) Erfinder: Abele, Tobias, 73568 Durlangen-Zimmerbach (DE); Telles, Hans, 71384 Weinstadt (DE)
(74) Vertreter: Thum, Bernhard

(56) Entgegenhaltungen:
- EP-A1- 2 070 495
- EP-A1- 2 502 608
- EP-A2- 1 743 608
- EP-A2- 1 834 613
- EP-B1- 2 070 495
- EP-B1- 2 502 608
- EP-B9- 1 834 613
- WO-A1-2013/106666
- DE-A1-102012 013 386
- DE-U1- 29 720 475
- US-A1- 2012 022 420
- US-A1- 2015 366 697
- medi GmbH & Co. KG: "Spinomed Rückenorthese anlegen | Anleitung für medizinisches Personal & Techniker", , 9 April 2013 (2013-04-09), page 1, XP054982327, Retrieved from the Internet: URL:https://www.youtube.com/watch?v=PEEtM9 BLc5Ihttps://www.youtube.com/watch?v=PEEtM 9BLc5I [retrieved on 2021-10-07]

## Beschreibung

Die vorliegende Erfindung betrifft eine Rückenorthese zum Stabilisieren des Rückens eines Patienten und ein Verfahren zum Anlegen einer solchen Rückenorthese.

Bei Rückenerkrankungen kann eine Krümmung der Wirbelsäule eines Patienten auftreten. Diese kann entweder angeboren sein, aufgrund von Erkrankungen oder durch übermäßige Belastung des Patienten verursacht sein. Derartige Erkrankungen können stabile, osteoporotische Wirbelkörperfrakturen, Osteoporose, sekundäre Kyphosen, wie stabile Frakturen oder Tumore, muskuläre Insuffizienz und andere Krankheitsbilder sein. Die Krümmung der Wirbelsäule kann im Verlauf der Krankheit zunehmen. Der Verlauf einer solchen Erkrankung kann über einen langen Zeitraum erfolgen.

Um derartige Erkrankungen zu therapieren, sind neben chirurgischen Eingriffen auch konservative Methoden bekannt, die den Einsatz von stützenden Vorrichtungen vorsehen. Derartige Vorrichtungen stabilisieren den Rücken des Patienten und wirken wirbelsäulenaufrichtend.

Aus EP 0 917 864 B1 ist beispielsweise eine derartige Rückenorthese zum Stabilisieren des Rückens eines Patienten bekannt. Diese Rückenorthese umfasst mehrere Komponenten, nämlich eine Rückenschiene, die am Rücken des Patienten anliegt. Ferner umfasst diese Gurte, um die Rückenschiene an dem Patienten zu befestigen und zusammen mit der Rückenschiene den Oberkörper und die Wirbelsäule des Patienten wieder aufzurichten. Zu den Gurten gehören Schultergurte und Hüftgurte. Ein Schultergurt verläuft dabei üblicherweise von dem Rückenelement über eine Schulter eines Patienten zur Vorderseite des Patienten, wird an einem mittleren Bereich der Rückenschiene umgelenkt und ist schließlich an einem Hüftgurt befestigt.

EP 1 743 608 A2 offenbart eine Rückenorthese mit einem sich entlang eines Rückens erstreckenden Stützteils, einer in einem Abdominalbereich eines Patienten zu tragenden Bandage, die über einen Verschluss verschließbar ist, und einer Gurtanordnung. An dem Stützteil ist ein Ende eines unteren Gurtes befestigt, wobei der untere Gurt an dessen oberen Ende mit einer Schnalle mit der Gurtanordnung verbunden ist.

US 2015/366 697 A1 und US 2012/022 420 offenbaren jeweils eine Rückenorthese mit einem zweiteiligen Schultergurt. Ein oberer Schultergurt ist über eine Schnalle mit einem unteren Gurtabschnitt verbunden, der mit einem Rückenelement verbunden ist.

Weitere Rückenorthesen sind aus Dokumenten EP 1 834 613 A2 und DE 297 20 475 U1 bekannt.

Ferner offenbart das Dokument WO 2013/106666 verschiedene Rückenorthesen, die an die Bedürfnisse des Patienten anpassbar sind. Beispielsweise sind gemäß einer Ausführungsform die beiden Schultergurtzüge zweiteilig ausgeführt, wodurch die Länge der oberen Schultergurtzüge einstellbar ist. Diese Ausführungsform offenbart die Merkmale des Oberbegriffs des Anspruchs 1.

Üblicherweise werden bekannte Rückenorthese von einem Arzt oder Therapeuten verordnet. Die anatomischen Gegebenheiten der Patientenrücken und die pathologischen Ausprägungen von Rückenerkrankungen können sich allerdings stark unterscheiden. Es ist folglich notwendig, dass die betreffende Osteoporose-Orthese an den Rücken des Patienten angepasst wird und dabei die individuellen Gegebenheiten eines Patienten berücksichtigt werden. Daher ist bekannt, dass eine geschulte Person aus einer Vielzahl von Rückenorthesen ein geeignetes Produkt auswählt und dieses darüber hinaus speziell an den Patienten anpasst. Ferner ist bekannt, dass Rückenorthesen individuell für einen Patienten hergestellt werden.

Die aus dem Stand der Technik bekannten vorgefertigten Rückenorthesen sind allerdings in ihrer Anpassbarkeit an den Patienten beschränkt. Die Anpassung an den Patienten - sofern überhaupt möglich - ist mit viel Aufwand verbunden und erfordert geschultes Personal. Auch die Anpassung derartiger Orthesen während der Therapie ist durch deren geringe Anpassbarkeit eingeschränkt, sodass oftmals im Therapieverlauf vollständig neue Orthesen angefertigt werden müssen.

Die Aufgabe der vorliegenden Erfindung ist es daher, eine Rückenorthese zum Stabilisieren des Rückens eines Patienten bereitzustellen, die entsprechend den Erfordernissen eines Patienten eingestellt werden kann. Ferner ist es Aufgabe der vorliegenden Erfindung, ein Verfahren zum Anlegen einer Rückenorthese bereitzustellen.

Die erfindungsgemäße Aufgabe wird durch eine Rückenorthese zum Stabilisieren des Rückens eines Patienten nach Anspruch 1 gelöst. Ferner wird die erfindungsgemäße Aufgabe durch ein Verfahren zum Anlegen einer Rückenorthese nach Anspruch 15 gelöst. Vorteilhafte Weiterbildungen der Erfindung sind in den Unteransprüchen beschrieben.

Die oben genannte Aufgabe wird gemäß Anspruch 1 durch eine Rückenorthese zum Stabilisieren des Rückens eines Patienten gelöst, die ein Rückenelement mit einem Stabilisierungskörper umfasst, das den Rücken abstützt. Ferner umfasst die erfindungsgemäße Rückenorthese eine mit dem Rückenelement verbundene Hüftgurtanordnung zur hüftnahen Anbringung am Patienten, sowie eine Schultergurtanordnung mit wenigstens zwei Schultergurtzügen. Jeder Schultergurtzug wird bei Anbringung am Patienten von dem Rückenelement über eine Schulter des Patienten durch wenigstens ein an dem Rückenelement angeordnetes Umlenkorgan zur Hüftgurtanordnung geführt und ist mit der Hüftgurtanordnung koppelbar oder gekoppelt. Ferner sind die beiden Schultergurtzüge zweiteilig. Die beiden Schultergurtzüge umfassen mindestens einen oberen Schultergurtabschnitt, der sich von dem Rückenelement bis über die Schultern erstreckt, und mindestens einen unteren Schultergurtabschnitt, der sich von dem oberen Schultergurtabschnitt über das an dem Rückenelement angeordnete Umlenkorgan bis zur Hüftgurtanordnung erstreckt. Der mindestens eine obere Schultergurtabschnitt eines der beiden Schultergurtzüge und der mindestens eine untere Schultergurtabschnitt dieses Schultergurtzugs sind über ein Verbindungselement miteinander verbindbar. Ferner ist die Länge wenigstens eines der Schultergurtzüge einstellbar.

Die Zweiteiligkeit der beiden Schultergurtzüge erleichtert das Anlegen der Rückenorthese und das Einstellen an die individuellen Gegebenheiten eines Patienten. Ferner kann es vorteilhaft sein, beide Abschnitte unabhängig voneinander auszugestalten. Liegt ein Abschnitt beispielsweise direkt an dem Körper des Patienten an, so kann dieser Abschnitt mit Polstern versehen sein. Dies erhöht den Tragekomfort der Rückenorthese.

Ferner weist die Ausgestaltung mit oberem und unterem Schultergurtabschnitt den Vorteil auf, dass der jeweilige obere und der untere Schultergurtabschnitt unabhängig voneinander ausgelegt werden können bzw. beschaffen sein können. Beispielsweise kann der obere Schultergurtabschnitt verbreitert ausgeführt sein und/oder Polster aufweisen. Dies kann den Tragekomfort der Rückenorthese erhöhen. Ferner kann der untere Schultergurtabschnitt verschmälert ausgeführt sein. Dies kann die Bewegungsfreiheit eines Patienten erhöhen. Insgesamt kann ein Tragen und Anpassen der Rückenorthese ähnlich einem Rucksack ermöglicht werden.

Das Verbindungselement kann das Anlegen der Rückenorthese und das Einstellen der Rückenorthese an den Patienten erleichtern. Beispielsweise kann das Verbindungselement fest mit dem oberen oder unteren Schultergurtabschnitt verbunden sein, während es mit dem jeweils anderen Schultergurtabschnitt lösbar verbunden ist. Dies erhöht die Einstellbarkeit der Rückenorthese.

Die Einstellbarkeit der Länge wenigstens eines der Schultergurtzüge ermöglicht eine vorteilhafte Anpassbarkeit der Rückenorthese an die jeweiligen anatomischen und pathologischen Gegebenheiten eines Patienten. Dabei ist es beispielsweise möglich, die Länge der Schultergurte an die Größe eines Patienten anzupassen. Ferner ist es beispielsweise möglich, die Länge der Schultergurte an das Ausmaß der Rückenkrümmung des Patienten anzupassen. Darüber hinaus kann in einem Zustand, in dem die Rückenorthese nicht angelegt ist, die Länge der Schultergurtzüge länger sein als in einem getragenem Zustand. Ein Patient kann damit einfacher einen Schultergurtzug greifen und über eine Schulter legen. In einem folgenden Schritt kann die Länge eines Schultergurtzugs verkürzt werden, sodass sich die Rückenorthese insgesamt enger und straffer am Körper des Patienten anlegt. Dadurch kann das Anlegen der Rückenorthese durch den Patienten ohne zusätzliche Hilfe erleichtert werden. Gleichzeitig bleibt die therapeutische Wirkung in dem angelegten Zustand der Rückenorthese erhalten und kann bei Bedarf auch vom Patienten verändert werden, beispielsweise um eine zu straffe Einstellung etwas zu lockern.

Insgesamt ist mit der erfindungsgemäßen Rückenorthese ein einfaches und komfortables Einstellen der Rückenorthese bzw. der Länge der Schultergurtzüge der Schultergurtanordnung möglich. Das Anlegen und Einstellen der Rückenorthese kann dabei ähnlich wie bei einem Rucksack erfolgen. Das An- und Ablegen der Rückenorthese wird erleichtert. Es kann durch das Einstellen der Länge der Schultergurtzüge die Reklination bzw. die Aufrichtung des Patienten ohne großen Kraftaufwand angepasst werden, in dem die Zügelstärke variiert wird. Durch die anpassbare Reklination kann die Atmung des Patienten freier werden. Der Tragekomfort einer Rückenorthese kann durch die erfindungsgemäße Ausgestaltung erhöht werden. Die Rückenorthese kann an individuelle Vorlieben eines Patienten, an anatomische und pathologische Gegebenheiten oder an eine Therapie eines Patienten angepasst werden.

Ferner kann erfindungsgemäß vorgesehen sein, dass das Verbindungselement ein Umlenkverschluss, beispielsweise in Form einer Schnalle sein. Durch den Umlenkverschluss ist es möglich, dass die Länge des oberen oder unteren Schultergurtabschnitts anpassbar ist. Durch den Umlenkverschluss kann die umgelenkte Länge des jeweiligen unteren und/oder oberen Schultergurtabschnitts eingestellt werden. Damit ist der entsprechende untere und/oder obere Schultergurtabschnitt in seiner Länge veränderbar. So ist die Rückenorthese leicht anlegbar und einfach einstellbar. Im Fall der Einstellbarkeit des unteren Schultergurtabschnitts kann bei dessen Einstellung der obere Schultergurtabschnitt unverändert bleiben. Dennoch kann die Länge eines Schultergurtzugs eingestellt werden. Liegt dabei ferner der obere Schultergurtabschnitt direkt an der Schulter bzw. an dem Körper des Patienten an und liegt der untere Schultergurtabschnitt nur teilweise am Körper des Patienten an, so kann die Länge des Schultergurtzugs eingestellt werden, ohne dass in unerwünschter Weise Kleidung oder Haut eines Patienten in dem Verbindungselement eingeklemmt wird.

Gemäß der vorliegenden Erfindung nach Anspruch 1 ist die Position des Umlenkorgans relativ zu dem Rückenelement veränderbar. Dadurch ist es möglich, dass der Verlauf eines Schultergurtzugs und damit die Rückenorthese an die Erfordernisse eines Patienten einfach angepasst werden kann. Der Tragekomfort für einen Patienten kann gesteigert werden. Eine bessere Anpassung an die Anatomie des Patienten sowie an das Ausmaß der Wirbelsäulenkrümmung ist erfindungsgemäß einfach möglich. So kann die therapeutische Wirkung der Rückenorthese gesteigert werden. Die Rückenorthese trägt wenig auf und ist leicht und angenehm zu tragen. Ferner kann die Reklination des Patienten gesteigert werden, sodass dessen Atmung freier wird. Weiterhin ermöglicht die erfindungsgemäße Rückenorthese, dass der Oberkörper eines Patienten muskulär aktiviert und aufgerichtet wird. Der Rücken des Patienten kann entlastet und gleichzeitig stabilisiert werden. Schulter und Arme des Patienten bleiben frei beweglich. Die Mobilität eines Patienten kann gesteigert werden.

Eine Weiterbildung der Erfindung sieht vor, dass die Rückenorthese ferner eine Rückenpelotte umfasst, an der das wenigstens eine Umlenkorgan angebracht ist. Die Rückenpelotte kann dabei als eine Art Polster ausgebildet sein. Über diese Rückenpelotte kann eine Kraft von dem Umlenkorgan auf das Rückenelement übertragen werden. Bevorzugt ist die Rückenpelotte auf Höhe des unteren Rippenbogens positioniert. Die Krafteinleitung in das Rückenelement kann somit über die Rückenpelotte gezielt erfolgen.

Ferner kann erfindungsgemäß vorgesehen sein, dass an der Rückenpelotte wenigstens ein Umlenkgurt vorgesehen ist, an dem das wenigstens eine Umlenkorgan angebracht ist. Durch Verwendung eines Umlenkgurts ist es möglich, die Position des Umlenkorgans zu beeinflussen. Damit kann der Gurtverlauf gezielt eingestellt werden. Beispielsweise kann während einem Produktionsprozess oder bereits bei der Entwicklung der Rückenorthese die Länge des Umlenkgurtes gezielt ausgewählt und damit die Position des Umlenkorgangs beeinflusst werden. Auch lassen sich je nach Bedarf unterschiedlich lange Umlenkgurte bereitstellen, beispielsweise in kleiner, mittlerer und großer Länge.

In einer bevorzugten erfindungsgemäßen alternativen Ausgestaltung ohne Rückenpelotte kann ferner vorgesehen sein, dass an dem Rückenelement wenigstens ein Umlenkgurt angebracht ist, an dem das wenigstens eine Umlenkorgan angebracht ist. So ist es möglich, dass eine Kraft ausgehend von dem Umlenkorgan über den Umlenkgurt gezielt in das Rückenelement eingeleitet werden kann. Ferner ist es möglich, die Position des wenigstens einen Umlenkgurts gegenüber dem Rückenelement anzupassen. Dies kann entweder bereits während einem Produktionsprozess und bei der Entwicklung der Rückenorthese erfolgen, sodass die Position des Umlenkgurts nach der Produktion festgelegt ist. Auch kann die Positionierung des Umlenkgurts gegenüber dem Rückenelement anpassbar gestaltet sein. Der Umlenkgurt kann beispielsweise über eine Verschiebevorrichtung gegenüber dem Rückenelement verschieblich ausgebildet sein. Bevorzugt ist der Umlenkgurt auf Höhe des unteren Rippenbogens des Patienten mit dem Rückenelement verbunden. Insgesamt ist eine gegenüber dem Stand der Technik verbesserte Anpassung an die anatomischen und pathologischen Gegebenheiten des betreffenden Patienten möglich. Dadurch können sowohl der Tragekomfort als auch die therapeutische Wirkung der Rückenorthese gesteigert werden.

Gemäß einer bevorzugten Ausführungsform der Erfindung kann der wenigstens eine Umlenkgurt in dessen Länge veränderbar sein. Dadurch ist es möglich, dass die Position des Umlenkorgans relativ zum Patienten eingestellt werden kann. Dies kann im Rahmen des Therapieverlaufs vorteilhaft sein, da damit der Verlauf eines Schultergurtzugs beeinflusst werden kann. Eine ergonomische Einstellung und therapiebedingte Nachstellung der Rückenorthese ist damit möglich. Der Tragekomfort für den Patienten kann gesteigert werden. Weiterhin bewirkt die veränderbare Länge des Umlenkgurts einen veränderbaren Kraftfluss.

Ferner kann erfindungsgemäß vorgesehen sein, dass die Rückenpelotte mit dem Rückenelement verlagerbar verbunden ist. Dies kann insbesondere über einen Verschiebemechanismus oder einen Klettverschluss erfolgen. Der Verschiebemechanismus kann dabei derart ausgebildet sein, dass die Rückenpelotte dauerhaft mit dem Rückenelement verbunden bleibt, und dennoch gegenüber dem Rückenelement verschoben werden kann. Ein Klettverschluss zur Verbindung von Rückenpelotte und Rückenelement kann dazu dienen, dass die Rückenpelotte von dem Rückenelement getrennt und nach einer Neupositionierung erneut mit dem Rückenelement verbunden werden kann. Die Rückenorthese ist damit flexibel und an individuelle Gegebenheiten anpassbar. Ferner ist die Verlagerbarkeit besonders vorteilhaft, da damit der Ort der Krafteinleitung von der Rückenpelotte in das Rückenelement angepasst werden kann. Eine an den Patienten angepasste, die Therapie unterstützende Krafteinleitung ist damit möglich. Bevorzugt ist die Rückenplatte auf Höhe des unteren Rippenbogens des Patienten positioniert.

Gemäß Anspruch 1 ist die Schultergurtanordnung mit der Hüftgurtanordnung über mindestens ein Führungselement verbunden. Durch die Ausgestaltung des Führungselements kann damit die Krafteinleitung einer Kraft zwischen Schultergurtanordnung und Hüftgurtanordnung gesteuert werden.

Gemäß einer erfindungsgemäßen Ausführungsvariante kann die Ausrichtung des Führungselements relativ zu der Hüftgurtanordnung veränderbar sein. Eine individuelle Anpassung an die jeweiligen anatomischen Gegebenheiten von Patienten ist damit möglich. Beispielsweise wird bei einem dünneren Patienten das Führungselement und die damit verbundene Schultergurtanordnung eher in Richtung Bauchmitte orientiert sein, während bei einem kräftiger oder fülliger ausgebildeten Patienten das Führungselement und die damit verbundene Schultergurtanordnung tendenziell eher in Richtung einer Flanke eines Patienten bzw. eher lateral ausgerichtet werden kann.

Gemäß einer Ausführungsform kann das Führungselement an der Hüftgurtanordnung anbringbar oder angebracht und relativ zu der Hüftgurtanordnung verschwenkbar sein. Ist das Führungselement wahlweise an der Hüftgurtanordnung anbringbar, so kann dies das Anlegen der Rückenorthese erleichtern. In diesem Zusammenhang kann vorgesehen sein, dass das Führungselement zunächst von der Hüftgurtanordnung gelöst ist und nach dem Anlegen der Rückenorthese zum Tragen der Rückenorthese an der Hüftgurtanordnung befestigt wird. Ist das Führungselement verschwenkbar, so kann sich die Orientierung des Führungselements an die anatomischen Gegebenheiten eines Patienten anpassen. Beispielsweise wird bei einem dünneren Patienten das Führungselement und die damit verbundene Schultergurtanordnung eher in Richtung Bauchmitte orientiert sein, während bei einem kräftigeren oder fülligeren Patienten das Führungselement und die damit verbundene Schultergurtanordnung tendenziell eher in Richtung einer Flanke eines Patienten bzw. eher lateral ausgerichtet ist.

Eine weitere erfindungsgemäße Ausgestaltung sieht vor, dass die Hüftgurtanordnung in ihrer Länge einstellbar ist. Damit ist die Rückenorthese an unterschiedliche Patientenkörper individuell anpassbar. Beispielsweise erfordert ein kräftiger oder völliger Patient eine längere Hüftgurtanordnung als ein dünnerer Patient. Eine Rückenorthese kann dabei an unterschiedliche Bauchumfänge von Patienten angepasst werden. Es können unterschiedlich lange Hüftgurtanordnungen bereitgestellt werden.

Gemäß einer weiteren Ausgestaltung der Erfindung kann vorgesehen sein, dass die Hüftgurtanordnung eine Bauchpelotte umfasst, die mit der Schultergurtanordnung koppelbar oder gekoppelt ist. Die Bauchpelotte kann für eine großflächige, aber auch eine gezielte Krafteinleitung in den Bauchbereich des Patienten dienen. Die Koppelbarkeit von Schultergurtanordnung und Bauchpelotte kann das Anlegen der Rückenorthese erleichtern. Bevorzugt ist die Bauchpelotte auf Höhe des Schambeins des Patienten positioniert.

Ferner kann erfindungsgemäß vorgesehen sein, dass die Bauchpelotte bei Anbringung an einem Patienten an seiner Vorderseite angeordnet ist. Damit ist die Bauchpelotte für einen Patienten leicht zu greifen. Bevorzugt ist die Bauchpelotte auf Höhe des Schambeins des Patienten positioniert.

Weiter kann erfindungsgemäß vorgesehen sein, dass die Bauchpelotte mehrteilig ausgebildet ist, sodass diese mindestens ein erstes und ein zweites Bauchpelottenelement umfasst, die miteinander verbindbar und voneinander trennbar sind. Dies birgt den Vorteil, dass die Bauchpelotte einfach geöffnet bzw. geschlossen werden kann. Folglich kann die Hüftgurtanordnung einfach an den Patienten angelegt und am Patienten gelöst werden. Dies kann der Patient selbst ohne großen Aufwand oder alternativ eine andere Person durchführen. Beispielsweise sind in einem geöffneten Zustand das erste und das zweite Bauchpelottenelement voneinander getrennt, sodass sich der Hüftgurt in einem geöffneten Zustand befindet. Nach dem Anlegen an den Patienten sind das erste und das zweite Bauchpelottenelement miteinander verbunden, sodass sich der Hüftgurt in einem geschlossenen Zustand befindet. Insgesamt lässt sich die Rückenorthese einfach an- bzw. ablegen. Ferner kann ein Patient die Rückenorthese mit geringem Kraftaufwand selbstständig anlegen bzw. ablegen.

Eine Weiterbildung der Erfindung sieht vor, dass das erste und zweite Bauchpelottenelement über einen Klettverschluss lösbar miteinander verbunden sind. Dies vereinfacht das Verbinden und Trennen des ersten und zweiten Bauchpelottenelements. Dadurch lassen sich der Hüftgurt und damit die Rückenorthese einfacher an- bzw. ablegen. Weiterhin kann vorgesehen sein, dass die Verschlussposition der Bauchpelottenelemente variiert werden kann. Folglich können die beiden Bauchpelottenelemente in einer Vielzahl von relativen Positionen zueinander miteinander verbunden werden. So kann die Länge des Hüftgurts einfach an die Erfordernisse des Patienten angepasst werden.

Gemäß einer weiteren Ausführungsform der Erfindung kann die Hüftgurtanordnung wenigstens ein Hüftband umfassen, das sich von dem Rückenelement aus zu der Bauchpelotte hin erstreckt und dessen Länge einstellbar ist. Folglich kann der Hüftgurt individuell durch Einstellung der Länge des Hüftbands an die anatomischen Gegebenheiten und/oder an die therapeutischen Erfordernisse und/oder an die Vorlieben eines Patienten angepasst werden. Es ist beispielsweise gemäß dieser Ausführungsform auch möglich, dass zwischen dem Rückenelement und jeder der beiden Bauchpelottenelemente ein Hüftband vorgesehen ist. So ist die Hüftgurtanordnung einfacher und genauer an den Patienten anpassbar. Der Tragekomfort des Patienten kann gesteigert werden.

Ferner kann ein Hüftband einen Klettverschluss aufweisen. Dies kann ein sogenannter Krokodil-Klettverschluss sein. Dies ermöglicht die einfache, gezielte und korrekte Einstellung der Rückenorthese. Ein Verrutschen der Orthese zum Oberkörper hin wird verhindert.

Eine Weiterbildung der Erfindung sieht vor, dass die Hüftgurtanordnung zwei Hüftbänder umfasst, wobei jeweils ein Hüftband und jeweils einer der Schultergurtzüge an einem Bauchpelottenelement anbringbar oder angebracht ist. Damit ist es möglich, dass die Rückenorthese einfach von einem Patienten selbst an dessen Körper an- bzw. abgelegt werden kann.

Ferner kann erfindungsgemäß vorgesehen sein, dass sich der Stabilisierungskörper im Wesentlichen entlang der Wirbelsäule des Patienten erstreckt. Damit kann der Rücken eines Patienten großflächig stabilisiert bzw. abgestützt werden. Andererseits kann die Rückenorthese dadurch gezielt auf die Wirbelsäule des Patienten einwirken. Es ist allerdings auch möglich, dass der Stabilisierungskörper sich nur entlang eines Teils oder entlang von Teilen der Wirbelsäule erstreckt. Eine gezielte Unterstützung der Wirbelsäule in zu unterstützenden oder zu therapierenden Bereichen wird dadurch ermöglicht.

Weiterhin kann vorgesehen sein, dass der Stabilisierungskörper fest oder flexibel anpassbar ist. Bevorzugt ist der Stabilisierungskörper in einem Bearbeitungszustand verformbar und an den Rücken eines Patienten anpassbar, während er in einem getragenen Zustand fest ist. Der Stabilisierungskörper kann in Form einer deformierbaren Aluminiumschiene ausgebildet sein. Auch andere Materialien können vorteilhaft sein denkbar, beispielsweise Metalle, Kunststoffe, Faserverbundwerkstoffe, natürliche Werkstoffe wie Holz etc. Auch eine Kombination mehrerer Werkstoffe ist möglich.

Der Stabilisierungskörper kann als ein Element in Form einer Schiene ausgebildet sein. Auch ist es möglich, dass der Stabilisierungskörper in Form eines Körpers mit verschiedenen Segmenten ausgebildet ist. Diese Segmente können miteinander fest oder über Gelenke miteinander verbunden sein.

Es kann erfindungsgemäß vorgesehen sein, dass der Stabilisierungskörper in verschiedenen Längen vorliegt. Je nach Größe des Patienten kann ein passender Stabilisierungskörper ausgewählt werden. Der Stabilisierungskörper kann eine anatomische Formgebung aufweisen oder in diese gebracht werden.

Der Stabilisierungskörper kann Elemente aufweisen, die in ihrer Struktur perforiert sind bzw. Hohlräume aufweisen. Damit kann ein geringeres Gewicht des Stabilisierungskörpers erreicht werden.

Ferner wird die eingangs bezeichnete der Erfindung zugrunde liegende Aufgabe durch ein Verfahren zum Anlegen einer Rückenorthese gelöst, wobei folgende Schritte durchgeführt werden. Zunächst wird die Schultergurtanordnung über die Schultern des Patienten gelegt. Anschließend wird der Rumpf des Patienten mit der Hüftgurtanordnung umschlossen. In einem weiteren Schritt wird die Länge wenigstens eines der Schultergurtzüge eingestellt. Dies hat den Vorteil, dass die Schultergurtanordnung ähnlich wie die Gurte eines Rucksacks individuell entsprechend den Erfordernissen eingestellt werden können. Der Tragekomfort kann somit gesteigert werden. Ferner können individuelle anatomische Gegebenheiten, individuelle therapeutische Erfordernisse, Therapiestadien oder Vorlieben eines Patienten berücksichtigt werden.

Ferner kann das erfindungsgemäße Verfahren den Schritt des Einstellens der Position des Umlenkorgans gegenüber dem Rückenelement umfassen. Mit diesem Verfahren kann das Anlegen der Rückenorthese vereinfacht werden. Weiterhin wird die Anpassbarkeit der Rückenorthese an den Patienten erleichtert. Es können individuelle anatomische Gegebenheiten, individuelle therapeutische Erfordernisse, Therapiestadien oder Vorlieben eines Patienten berücksichtigt werden.

Die Erfindung wird im Folgenden beispielhaft anhand der beiliegenden Figuren erläutert. Es stellen dar:
- Fig. 1: eine schematische schräge Seitenansicht einer erfindungsgemäßen Rückenorthese;
- Fig. 2: eine schematische Rückansicht einer erfindungsgemäßen Rückenorthese;
- Fig. 3: eine schematische Vorderansicht einer erfindungsgemäßen Rückenorthese in geschlossenem Zustand;
- Fig. 4: eine schematische Vorderansicht einer erfindungsgemäßen variierten Rückenorthese in geschlossenem Zustand;
- Fig. 5: eine schematische Vorderansicht einer erfindungsgemäßen Rückenorthese in geöffnetem Zustand;
- Fig. 6a: eine schematische Detailansicht des Bereichs um das Verbindungselement;
- Fig. 6b: eine schematische Schnittansicht der Detailansicht nach Fig. 6a durch das Verbindungselement;
- Fig. 7: eine schematische Ansicht einer erfindungsgemäßen Rückenorthese an einem Patienten;

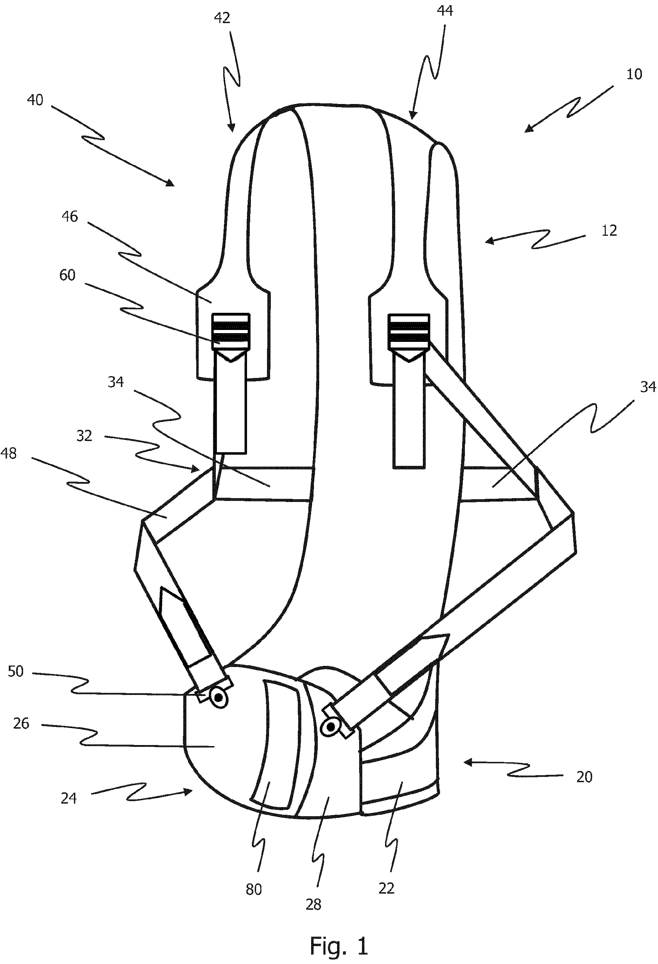

Figur 1 zeigt eine schematische schräge Seitenansicht einer erfindungsgemäßen Rückenorthese 10. Die Rückenorthese 10 umfasst ein Rückenelement 12 mit einem Stabilisierungskörper 14 (nicht dargestellt), eine Hüftgurtanordnung 20 und eine Schultergurtanordnung 40.

Die Schultergurtanordnung 40 weist zwei Schultergurtzüge 42, 44 auf. Ein Schultergurtzug 42, 44 erstreckt sich von dem Rückenelement 12 über eine Schulter eines Patienten durch ein Umlenkorgan 32 bis zur Hüftgurtanordnung 20. Jeder der Schultergurtzüge 42, 44 weist einen oberen Schultergurtabschnitt 46 und einen unteren Schultergurtabschnitt 48 auf, die jeweils über ein Verbindungselement 60 miteinander verbunden sind. Das Verbindungselement 60 ist eine Umlenkschnalle oder ein Umlenkverschluss. Der obere Schultergurtabschnitt 46 erstreckt sich von dem Rückenelement 12 über die Schulter eines Patienten bis zu dessen Vorderseite. Der untere Schultergurtabschnitt 48 verläuft von dem oberen Schultergurtabschnitt 46 bis zu der Hüftgurtanordnung 20. Der untere Schultergurtabschnitt 48 verläuft dabei durch das jeweilige Umlenkorgan 32. Das jeweilige Umlenkorgan 32 ist mit einem Umlenkgurt 34 verbunden. Der Umlenkgurt 34 verläuft von dem Umlenkorgan 32 zu einer Rückseite des Rückenelements 12.

Das Material der Schultergurtanordnung 40 mit den beiden Schultergurtzügen 42, 44 ist ein Gurtmaterial. Derartige Materialien werden beispielsweise bei Rucksäcken verwendet.

Die Hüftgurtanordnung 20 weist eine Bauchpelotte 24 auf. Die Bauchpelotte 24 umfasst ein erstes und ein zweites Bauchpelottenelement 26, 28. Die beiden Bauchpelottenelemente 26, 28 sind über einen Mechanismus miteinander verbindbar und voneinander trennbar. Dieser nicht näher dargestellte Mechanismus kann ein Klettverschluss sein. Das erste Bauchpelottenelement 26 weist eine Lasche 80 zum Greifen auf. Es ist auch möglich, dass beide Bauchpelottenelemente 26, 28 jeweils eine Lasche 80 zum Greifen aufweisen.

Die Hüftgurtanordnung 20 weist außerdem ein Hüftband 22 auf. Das Hüftband 22 verläuft von dem Rückenelement 12 bis zum ersten und zweiten Bauchpelottenelement 26, 28. In einer nicht dargestellten Ausführungsform kann das Hüftband 22 zweiteilig ausgebildet sein, sodass das eine Hüftband 22 von dem Rückenelement 12 zu dem ersten Bauchpelottenelement 26 und das andere Hüftband 22 von dem Rückenelement 12 zu dem zweiten Bauchpelottenelement 28 verläuft. Das Hüftband 22 kann über eine Schnalle mit einem Bauchpelottenelement 26, 28 verbunden sein. Die Länge des Hüftbands 22 kann durch Anpassen der umgelenkten Länge des Hüftbands 22 angepasst werden. Das Hüftband 22 kann einen Klettverschluss aufweisen, mit dem die eingestellte Länge des Hüftband 22 fixiert werden kann. Dabei greift der Klettverschluss auf dem Hüftband 22 selbst oder auf dem Rückenelement 12.

Das Material des Hüftbands 22 ist ein Gurtmaterial. Derartige Materialien werden beispielsweise bei Rucksäcken verwendet.

Der untere Schultergurtabschnitt 48 eines jeden Schultergurtzugs 42, 44 ist durch jeweils durch ein Führungselement 50 mit dem jeweiligen Bauchpelottenelement 26, 28 verbunden. Das Führungselement 50 ist eine Umlenkschnalle, sodass die Länge des unteren Schultergurtabschnitts 48 entsprechend einer Rucksackgurtverstellung eingestellt werden kann. Dieser Mechanismus kann ein Klettverschluss sein. In weiteren Ausführungsformen, die nicht zur Erfindung gehören, kann das Führungselement 50 ähnlich einer Gürtelschnalle mit Dorn ausgebildet sein. Durch Positionieren des Dorns in verschiedenen Löchern des unteren Schultergurtabschnitts 48 kann die Länge des unteren Schultergurtabschnitts 48 eingestellt werden. Ferner kann gemäß einer Alternative das Führungselement 50 derart ausgebildet sein, dass die Länge des unteren Schultergurtabschnitts 48 dauerhaft festgelegt ist.

Die Rückenorthese kann auf der Haut eines Patienten getragen werden. Ferner ist es möglich, dass die Rückenorthese auf einem geeigneten Kleidungsstück getragen wird.

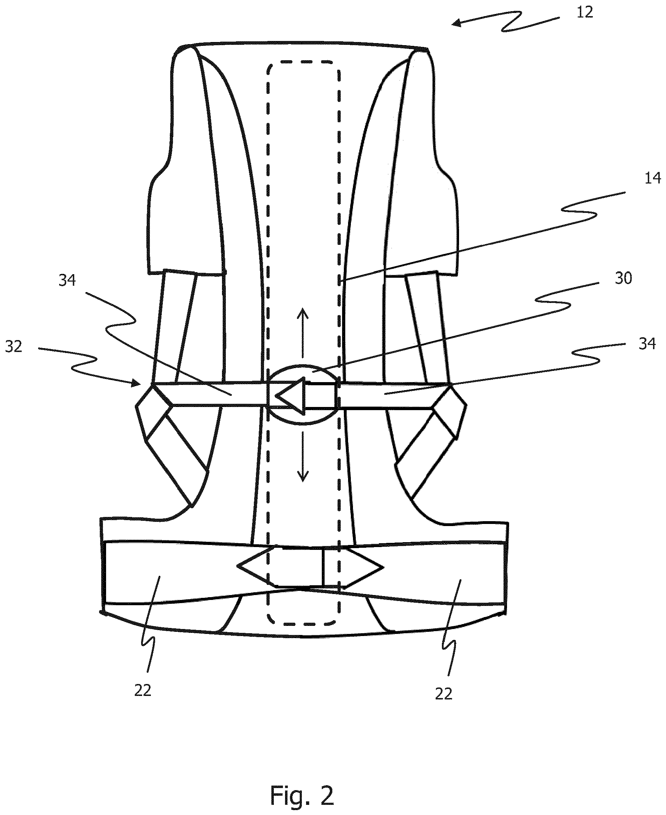

Figur 2 zeigt eine schematische Rückansicht einer erfindungsgemäßen Rückenorthese 10. Darin ist schematisch der Stabilisierungskörper 14 dargestellt. Der Stabilisierungskörper 14 liegt innerhalb des Rückenelements 12. Es ist jedoch auch möglich, dass der Stabilisierungskörper 14 außerhalb des Rückenelements 12 angeordnet ist. Der Umlenkgurt 34 bzw. die Umlenkgurte 34 sind mit einer Rückenpelotte 30 verbunden. Die Länge des Umlenkgurts 34 bzw. der Umlenkgurte 34 ist über einen Mechanismus einstellbar. Dieser Mechanismus kann ein Klettverschluss sein. Die Position der Rückenpelotte 30 ist gegenüber dem Rückenelement 12 und damit gegenüber dem Stabilisierungskörper 14 über einen Mechanismus veränderbar. Ein derartiger Mechanismus kann ein Klettverschluss sein. Die Rückenpelotte 30 ist im Wesentlichen mittig auf dem Rückenelement 12 angeordnet, sodass es zu einer linken und einer rechten Flanke eines Patienten in etwa gleich beanstandet ist.

Das Hüftband 22 ist bzw. die Hüftbänder 22 sind durch einen Mechanismus in dessen/deren Länge einstellbar. Ein derartiger Mechanismus kann ein Klettverschluss sein. Auch andere Mechanismen sind denkbar.

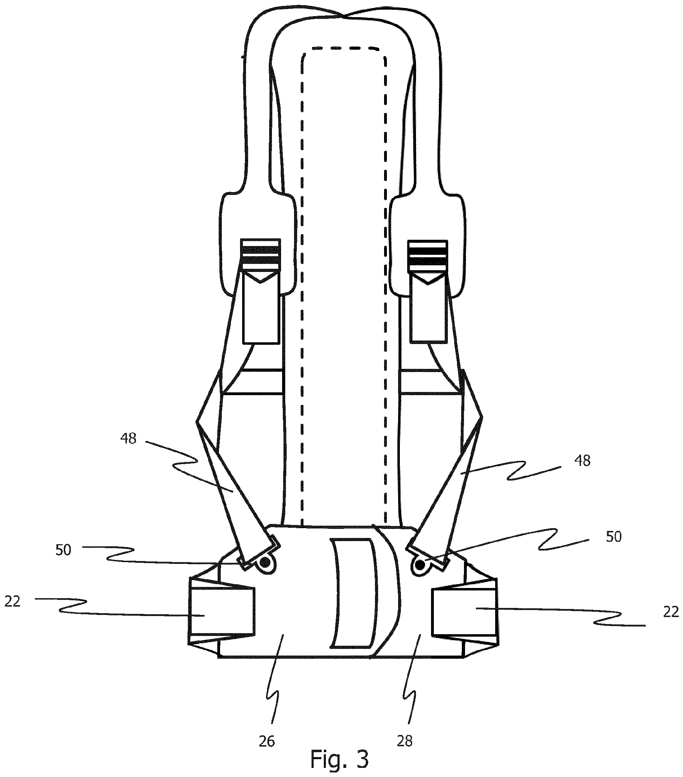

Figur 3 zeigt eine schematische Vorderansicht einer erfindungsgemäßen Rückenorthese 10 in geschlossenem Zustand. Die beiden Bauchpelottenelemente 26, 28 sind miteinander verbunden. Jedes Führungselement 50 verbindet den jeweiligen unteren Schultergurtabschnitt 48 drehbar mit dem jeweiligen Bauchpelottenelement 26, 28. Dadurch ist es möglich, dass sich die Position des jeweiligen unteren Schultergurtabschnitts 48 bzw. des jeweiligen Führungselements 50 relativ zu dem jeweiligen Bauchpelottenelement 26, 28 verändern lässt.

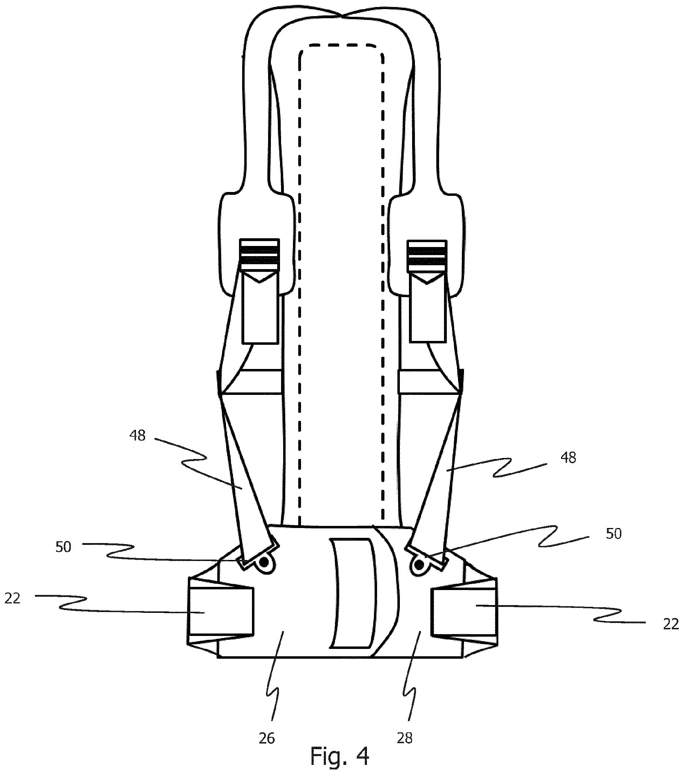

Figur 4 zeigt eine schematische Vorderansicht einer erfindungsgemäßen Rückenorthese 10 in geschlossenem Zustand mit variierter bzw. verkürzter Schultergurtanordnung 40. Gegenüber der in Figur 3 gezeigten Rückenorthese 10 ist der Verlauf des jeweiligen unteren Schultergurtabschnitts 48 verändert. Die jeweiligen Schultergurtabschnitte 48 verlaufen mehr in Richtung Mitte der Rückenorthese orientiert. Die jeweiligen Führungselemente 50 sind dadurch in ihrer Position relativ zu dem jeweiligen Bauchpelottenelement 26, 28 verdreht. Durch diese Drehbarkeit der Führungselemente 50 lässt sich die Schultergurtanordnung 40 an unterschiedliche Patienten anpassen. Die in Figur 4 gezeigte Rückenorthese 10 ist für einen dünneren Patienten angepasst, während die in Figur 3 gezeigte Rückenorthese 10 für einen kräftigeren Patienten angepasst ist.

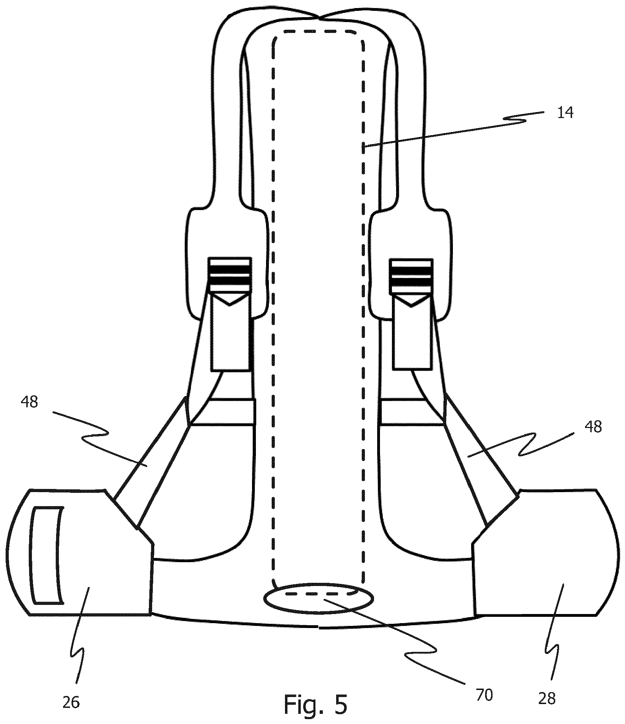

Figur 5 zeigt eine schematische Vorderansicht einer erfindungsgemäßen Rückenorthese 10 in geöffnetem Zustand. Die beiden Bauchpelottenelemente 26, 28 sind in dieser Ansicht voneinander getrennt. Dies erleichtert ein einfaches An- und Ablegen der Rückenorthese 10.

Das Material der Bauchpelottenelemente 26, 28 ist bevorzugt ein Gurtmaterial mit einer rauen, filzähnlichen Oberfläche. Dadurch ist es möglich, dass ein an mindestens einem Bauchpelottenelement 26, 28 angebrachter Klettverschluss mit dem anderen Bauchpelottenelement 26, 28 an verschiedenen Stellen verbunden und von diesem erneut getrennt werden kann, wodurch eine Einstellbarkeit erreicht wird. Auch andere Materialien für die Bauchpelottenelemente 26, 28 sind möglich. Entsprechende zusammengehörige Komponenten eines Klettverschlusses können auf der Oberfläche der Bauchpelottenelemente 26, 28 angebracht sein. Dies kann durch Aufnähen oder Aufkleben geschehen, oder durch Einhaken in entsprechende Hakenelemente oder durch Knopfverbindungen oder dergleichen.

Im unteren Bereich des Rückenelements 12 der Rückenorthese 10 ist ein Verschluss 70 angebracht. Dieser Verschluss 70 ist über einen Mechanismus lösbar mit dem Rückenelement 12 verbunden. Ein derartiger Mechanismus kann ein Klettverschluss sein. Der Verschluss 70 dient dazu, einen Zugang zum Inneren des Rückenelements 12 zu verschließen. Durch diesen Zugang kann der Stabilisierungskörper 14 in das Rückenelement 12 eingeführt und aus dem Rückenelement 12 erneut ausgeführt werden. Dies ermöglicht, dass der Stabilisierungskörper 14 herausgenommen, angepasst oder ausgetauscht und dann wieder eingelegt werden kann. Das Rückenelement 12 kann dabei aus einem Material mit hohem Tragekomfort sein. Das Rückenelement 12 kann aus verschiedenen Materialien zusammengesetzt sein. Dazu gehören beispielsweise Schaumstoffe und Filzmaterialien. Das Material des Rückenelements 12 ermöglicht ein angenehmes Tragen und verhindert ein direktes Anliegen des Stabilisierungskörpers 14 am Körper des Patienten.

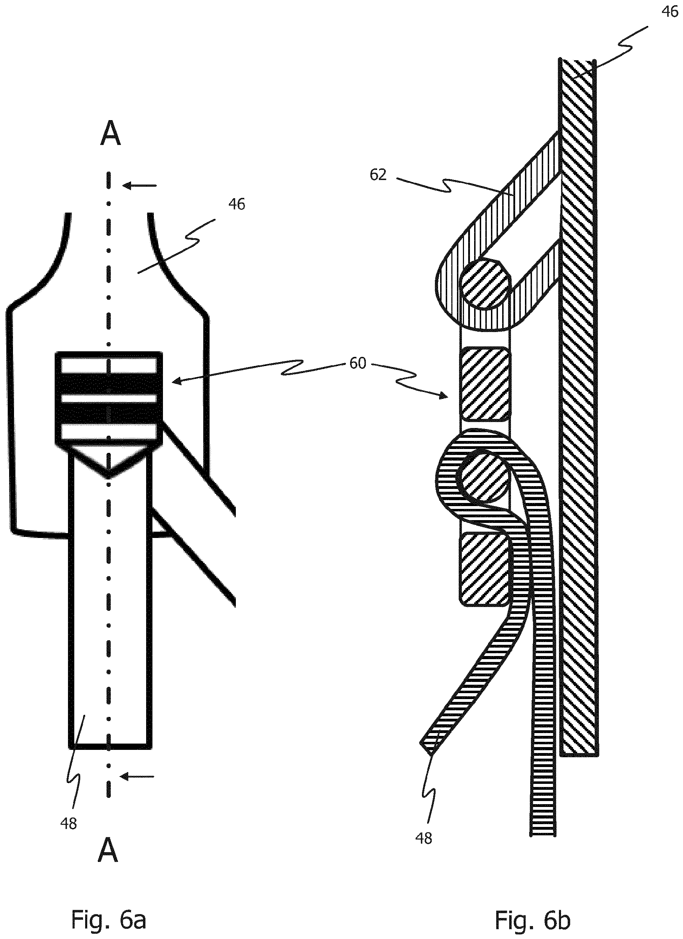

Figur 6a ist eine schematische Detailansicht des Bereichs um das Verbindungselement 60. Sie zeigt den oberen Schultergurtabschnitt 46 und den unteren Schultergurtabschnitt 48, die über das Verbindungselement 60 in Form einer Umlenkschnalle verbunden sind.

Figur 6b ist eine schematische Schnittansicht der Detailansicht nach Fig. 6a durch das Verbindungselement 60 entlang der Linie A-A. Es ist schematisch gezeigt, wie der obere Schultergurtabschnitt 46 mit dem unteren Schultergurtabschnitt 48 verbunden ist. Der obere Schultergurtabschnitt 46 ist über eine Gurtlasche 62 mit dem Verbindungselement 60 verbunden. Die Gurtlasche 62 umschließt dabei einen oberen Steg des Verbindungselements 60. Die beiden Enden der Gurtlasche 62 sind mit dem oberen Schultergurtabschnitt 46 dauerhaft verbunden. Dies kann durch Verkleben, Nähen oder andere Verbindungsformen geschehen. Der untere Schultergurtabschnitt 48 verläuft von einer unteren Seite ausgehend zwischen dem oberem Schultergurtabschnitt 46 und dem Verbindungselement 60, umschließt einen mittleren Steg des Verbindungselement 60, verläuft weiter zwischen einem unteren Steg des Verbindungselements 60 und dem oberen Schultergurtabschnitt 46 und endet in einem freien Bereich unterhalb des Verbindungselements 60.

Das Verbindungselement 60 besitzt durch die dargestellte Ausführung eine Klemmwirkung, die den unteren Schultergurtabschnitt 48 verklemmt und dadurch dessen Länge festlegt. Durch Ziehen am freien Ende des unteren Schultergurtabschnitts 48 kann die Länge des unteren Schultergurtabschnitts 48 verkürzt werden. Durch Anheben des Verbindungselements 60 in dessen unterem Bereich, also in einem Bereich um den unteren Steg das Verbindungselements 60, kann die Klemmwirkung des Verbindungselements 60 reduziert werden, sodass der untere Schultergurtabschnitt 48 verlängert werden kann. Dadurch wird das freie Ende des unteren Schultergurtabschnitts 48 kürzer. Das Verbindungselement 60 wirkt ähnlich wie ein Umlenkverschluss eines Rucksacks, mit dem die Länge der Gurte des Rucksacks eingestellt werden kann.

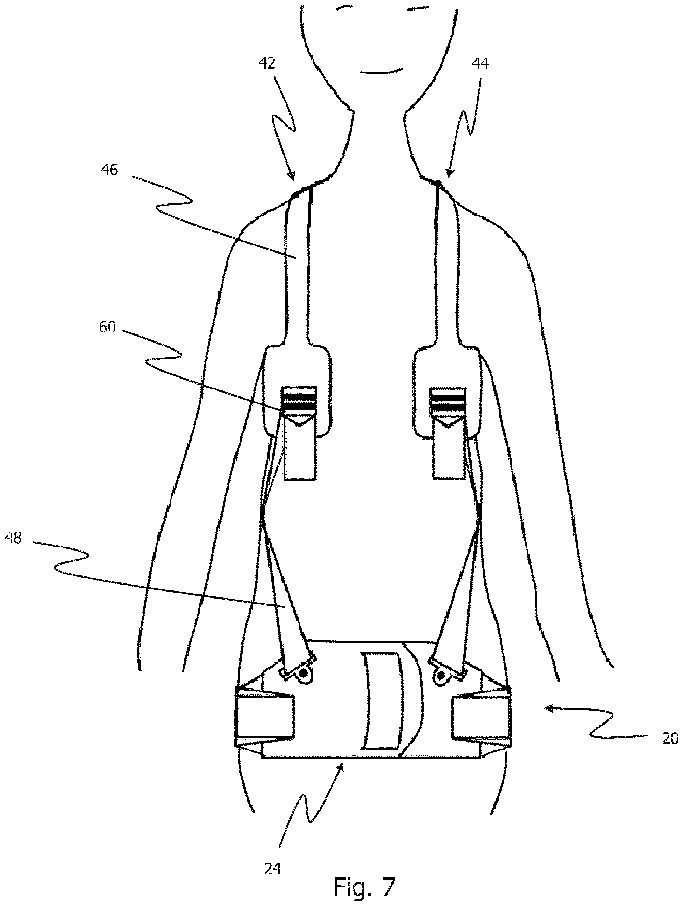

Figur 7 zeigt schematisch eine erfindungsgemäße Rückenorthese 10 in einem an einem Patienten angelegten Zustand. Die beiden Schultergurtzüge 42, 44 verlaufen über die Schultern des Patienten. Auf der Vorderseite ist der jeweilige obere Schultergurtabschnitt 46 mit dem unteren Schultergurtabschnitt 48 über das Verbindungselement 60 miteinander verbunden. Das Verbindungselement ist für den Patienten leicht greifbar. Diese Anordnung erlaubt ein rucksackähnliches Tragen und Anlegen der Rückenorthese 10. Die jeweiligen unteren Schultergurtabschnitte 48 sind mit der Bauchpelotte 24 der Hüftgurtanordnung 20 verbunden.

Zusammengefasst stellt die Erfindung eine Rückenorthese 10 bereit, die sich in relativ einfacher Weise an dem Patienten anlegen und auf dessen anatomische und pathologische Gegebenheiten individuell einstellen lässt.

## Patentansprüche

1. Rückenorthese (10) zum Stabilisieren des Rückens eines Patienten, umfassend:
ein Rückenelement (12) mit einem Stabilisierungskörper (14), das den Rücken abstützt,
eine mit dem Rückenelement (12) verbundene Hüftgurtanordnung (20) zur hüftnahen Anbringung am Patienten, und
eine Schultergurtanordnung (40) mit wenigstens zwei Schultergurtzügen (42, 44), wobei jeder Schultergurtzug (42, 44) bei Anbringung am Patienten von dem Rückenelement (12) über eine Schulter des Patienten durch wenigstens ein an dem Rückenelement (12) angeordnetes Umlenkorgan (32) zur Hüftgurtanordnung (20) geführt und mit der Hüftgurtanordnung (20) koppelbar oder gekoppelt ist,
wobei die beiden Schultergurtzüge (42, 44) zweiteilig sind,
wobei weiter die beiden Schultergurtzüge (42, 44) mindestens einen oberen Schultergurtabschnitt (46), der sich von dem Rückenelement (12) bis über die Schultern erstreckt, und mindestens einen unteren Schultergurtabschnitt (48) umfassen, der sich von dem oberen Schultergurtabschnitt (46) über das an dem Rückenelement (12) angeordnete Umlenkorgan (32) bis zur Hüftgurtanordnung (20) erstreckt, und
wobei der mindestens eine obere Schultergurtabschnitt (46) eines der beiden Schultergurtzüge (42, 44) und der mindestens eine untere Schultergurtabschnitt (48) dieses Schultergurtzugs (42, 44) über ein Verbindungselement (60) miteinander verbindbar sind, sodass die Länge wenigstens eines der Schultergurtzüge (42, 44) einstellbar ist, und
wobei die Schultergurtanordnung (40) mit der Hüftgurtanordnung (20) über mindestens ein Führungselement (50) verbunden ist,
**dadurch gekennzeichnet,**
**dass** das Führungselement (50) eine Umlenkschnalle ist, mittels der die Länge des unteren Schultergurtabschnitts (48) einstellbar ist, und
**dass** die Position des Umlenkorgans (32) relativ zu dem Rückenelement (12) veränderbar ist.

2. Rückenorthese (10) nach Anspruch 1, **dadurch gekennzeichnet, dass** das Verbindungselement (60) ein Umlenkverschluss ist, wobei bevorzugt durch den Umlenkverschluss die umgelenkte Länge des jeweiligen unteren Schultergurtabschnitts (48) eingestellt werden kann und damit der entsprechende untere Schultergurtabschnitt (48) in dessen Länge veränderbar ist.

3. Rückenorthese (10) nach einem der vorangehenden Ansprüche, ferner umfassend eine Rückenpelotte (30), an der das wenigstens eine Umlenkorgan (32) angebracht ist.

4. Rückenorthese (10) nach Anspruch 3, **dadurch gekennzeichnet, dass** an der Rückenpelotte (30) wenigstens ein Umlenkgurt (34) angebracht ist, an dem das wenigstens eine Umlenkorgan (32) angebracht ist.

5. Rückenorthese (10) nach einem der vorangehenden Ansprüche, **dadurch gekennzeichnet, dass** an dem Rückenelement (12) wenigstens ein Umlenkgurt (34) angebracht ist, an dem das wenigstens eine Umlenkorgan (32) angebracht ist.

6. Rückenorthese (10) nach einem der Ansprüche 4 oder 5, **dadurch gekennzeichnet, dass** der wenigstens eine Umlenkgurt (34) in dessen Länge veränderbar ist.

7. Rückenorthese (10) nach einem der Ansprüche 3, 5 oder 6, sofern auf Anspruch 5 rückbezogen, **dadurch gekennzeichnet, dass** die Rückenpelotte (30) mit dem Rückenelement (12) verlagerbar verbunden ist, insbesondere über einen Verschiebemechanismus oder einen Klettverschluss.

8. Rückenorthese (10) nach einem der vorhergehenden Ansprüche, **dadurch gekennzeichnet, dass** das Führungselement (50) an der Hüftgurtanordnung (20) anbringbar oder angebracht und relativ zu der Hüftgurtanordnung (20) verschwenkbar ist.

9. Rückenorthese (10) nach einem der vorhergehenden Ansprüche, **dadurch gekennzeichnet, dass** die Hüftgurtanordnung (20) in ihrer Länge einstellbar ist.

10. Rückenorthese (10) nach einem der vorhergehenden Ansprüche, **dadurch gekennzeichnet, dass** die Hüftgurtanordnung (20) eine Bauchpelotte (24) umfasst, die mit der Schultergurtanordnung (40) koppelbar oder gekoppelt ist, wobei bevorzugt die Bauchpelotte (24) mehrteilig ausgebildet ist, sodass diese mindestens ein erstes und ein zweites Bauchpelottenelement (26, 28) umfasst, die miteinander verbindbar und voneinander trennbar sind, wobei besonders bevorzugt das erste und zweite Bauchpelottenelement (26, 28) über einen Klettverschluss lösbar miteinander verbunden sind.

11. Rückenorthese (10) nach Anspruch 10, **dadurch gekennzeichnet, dass** die Hüftgurtanordnung (20) wenigstens ein Hüftband (22) umfasst, das sich von dem Rückenelement (12) aus zu der Bauchpelotte (24) hin erstreckt und dessen Länge einstellbar ist.

12. Rückenorthese (10) nach Anspruch 10, **dadurch gekennzeichnet, dass** die Hüftgurtanordnung (20) zwei Hüftbänder (22) umfasst, wobei jeweils ein Hüftband (22) und jeweils einer der Schultergurtzüge (42, 44) an einem Bauchpelottenelement (26, 28) anbringbar oder angebracht ist.

13. Rückenorthese (10) nach einem der vorhergehenden Ansprüche, **dadurch gekennzeichnet, dass** sich der Stabilisierungskörper (14) im Wesentlichen entlang der Wirbelsäule des Patienten erstreckt.

14. Verfahren zum Anlegen einer Rückenorthese (10) nach einem der Ansprüche 1 bis 13, wobei folgende Schritte durchgeführt werden:
- Legen der Schultergurtanordnung (40) über die Schultern des Patienten;
- Umschließen des Rumpfes des Patienten mit der Hüftgurtanordnung (20); und
- Einstellen der Länge wenigstens eines der Schultergurtzüge (42, 44).

## Claims

1. Back orthosis (10) for stabilizing a patient's back, comprising:
a back member (12) having a stabilizing body (14) and supporting the back,
a hip strap assembly (20) connected to the back member (12) for attaching said orthosis close to the patient's hips, and
a shoulder strap assembly (40) having at least two shoulder pull straps (42, 44), wherein each shoulder pull strap (42, 44), when being attached to the patient, is guided from the back member (12) to the hip strap assembly (20) over a shoulder of the patient and through at least one deflection member (32) arranged on the back member (12), and can be coupled or is coupled to the hip strap assembly (20),
wherein the two shoulder pull straps (42, 44) comprise two portions,
wherein the two shoulder pull straps (42, 44) further comprise at least one upper shoulder strap portion (46) that extends from the back member (12) over the shoulders, and at least one lower shoulder strap portion (48) that extends from the upper shoulder strap portion (46) to the hip strap assembly (20) via the deflection member (32) arranged on the back member (12), and
wherein the at least one upper shoulder strap portion (46) of one of the two shoulder pull straps (42, 44) and the at least one lower shoulder strap portion (48) of said shoulder pull strap (42, 44) are connectable to one another via a connecting member (60) so that the length of at least one of the shoulder pull straps (42, 44) is adjustable, and
wherein the shoulder strap assembly (40) is connected to the hip strap assembly (20) via at least one guide member (50),
**characterized in**
**that** the guide member (50) is a deflection buckle allowing to adjust the length of the lower shoulder strap portion (48), and
**that** the position of the deflection member (32) relative to the back member (12) is variable.

2. Back orthosis (10) according to claim 1, **characterized in that** the connecting member (60) is a deflection fastener, wherein the deflected length of the respective lower shoulder strap portion (48) can be adjusted preferably by means of the deflection fastener and thus the length of the corresponding lower shoulder strap portion (48) is variable.

3. Back orthosis (10) according to any one of the preceding claims, further comprising a back pad (30) to which the at least one deflection member (32) is attached.

4. Back orthosis (10) according to claim 3, **characterized in that** at least one deflection strap (34), to which the at least one deflection member (32) is attached, is attached to the back pad (30).

5. Back orthosis (10) according to any one of the preceding claims, **characterized in that** at least one deflection strap (34), to which the at least one deflection member (32) is attached, is attached to the back member (12).

6. Back orthosis (10) according to one of claims 4 or 5, **characterized in that** the length of the at least one deflection strap (34) is variable.

7. Back orthosis (10) according to one of claims 3, 5 or 6, if depending on claim 5, **characterized in that** the back pad (30) is displaceably connected to the back member (12), in particular by means of a sliding mechanism or a Velcro fastener.

8. Back orthosis (10) according to any one of the preceding claims, **characterized in that** the guide member (50) is attachable or attached to the hip strap assembly (20) and pivotable relative to the hip strap assembly (20).

9. Back orthosis (10) according to any one of the preceding claims, **characterized in that** the hip strap assembly (20) is adjustable in its length.

10. Back orthosis (10) according to any one of the preceding claims, **characterized in that** the hip strap assembly (20) comprises an abdominal pad (24) that can be coupled or is coupled to the shoulder strap assembly (40), the abdominal pad (24) preferably being formed of multiple parts so that it comprises at least a first and a second abdominal pad member (26, 28) that are connectable to and separable from one another, highly preferably the first and second abdominal pad members (26, 28) being detachably connected to one another by means of a Velcro fastener.

11. Back orthosis (10) according to claim 10, **characterized in that** the hip strap assembly (20) comprises at least one hip band (22) that extends from the back member (12) towards the abdominal pad (24) and whose length is adjustable.

12. Back orthosis (10) according to claim 10, **characterized in that** the hip strap assembly (20) comprises two hip bands (22), wherein one hip band (22) and one of the shoulder pull straps (42, 44) are attachable or attached to an abdominal pad member (26, 28).

13. Back orthosis (10) according to any one of the preceding claims, **characterized in that** the stabilizing body (14) extends substantially along the patient's spine.

14. Method of applying a back orthosis (10) according to any one of claims 1 to 13, comprising the steps of:
- placing the shoulder strap assembly (40) over the patient's shoulders;
- placing the hip strap assembly (20) around the patient's trunk; and
- adjusting the length of at least one of the shoulder pull straps (42, 44).

## Revendications

1. Orthèse dorsale (10) pour stabiliser le dos d'un patient, comprenant :
un élément dorsal (12) avec un corps stabilisateur (14) qui soutient le dos,
un ensemble ceinture de hanche (20) relié à l'élément dorsal (12) et destiné à être placé à proximité de la hanche du patient, et
un ensemble ceinture d'épaule (40) avec au moins deux harnais d'épaule (42, 44), chaque harnais d'épaule (42, 44) étant guidé, lors de la mise en place sur le patient, depuis l'élément dorsal (12) par une épaule du patient vers l'ensemble ceinture de hanche (20) en passant par au moins un organe de renvoi (32) disposé sur l'élément dorsal (12) et pouvant être accouplé ou étant accouplé à l'ensemble ceinture de hanche (20),
dans laquelle les deux harnais d'épaule (42, 44) sont en deux parties,
dans laquelle les deux harnais d'épaule (42, 44) comprennent en outre au moins une partie de ceinture d'épaule supérieure (46) qui s'étend depuis l'élément dorsal (12) jusqu'au-dessus des épaules, et au moins une partie de ceinture d'épaule inférieure (48) qui s'étend de la partie de ceinture d'épaule supérieure (46) jusqu'à l'ensemble ceinture de hanche (20) en passant par l'organe de renvoi (32) disposé sur l'élément dorsal (12), et
dans laquelle ladite au moins une partie de ceinture d'épaule supérieure (46) d'un des deux harnais d'épaule (42, 44) et ladite au moins une partie de ceinture d'épaule inférieure (48) de ce harnais d'épaule (42, 44) peuvent être reliées entre elles par un élément de liaison (60), de sorte que la longueur d'au moins un des harnais d'épaule (42, 44) est réglable, et
dans laquelle l'ensemble ceinture d'épaule (40) est relié à l'ensemble ceinture de hanche (20) par au moins un élément de guidage (50),
**caractérisée en ce que**
l'élément de guidage (50) est une boucle de renvoi au moyen de laquelle la longueur de la partie de ceinture d'épaule inférieure (48) est réglable, et **en ce que**
la position de l'organe de renvoi (32) par rapport à l'élément dorsal (12) est modifiable.

2. Orthèse dorsale (10) selon la revendication 1, **caractérisée en ce que** l'élément de liaison (60) est un fermoir de renvoi, le fermoir de renvoi permettant de préférence de régler la longueur déviée de la partie de ceinture d'épaule inférieure respective (48) et donc de modifier la longueur de la partie de ceinture d'épaule inférieure correspondante (48).

3. Orthèse dorsale (10) selon l'une des revendications précédentes, comprenant en outre une pelote dorsale (30) à laquelle ledit au moins un organe de renvoi (32) est fixé.

4. Orthèse dorsale (10) selon la revendication 3, **caractérisée en ce qu'**au moins une ceinture de renvoi (34) est fixée à la pelote dorsale (30), à laquelle ledit au moins un organe de renvoi (32) est fixé.

5. Orthèse dorsale (10) selon l'une des revendications précédentes, **caractérisée en ce qu'**au moins une ceinture de renvoi (34) est fixée à l'élément dorsal (12), à laquelle ledit au moins un organe de renvoi (32) est fixé.

6. Orthèse dorsale (10) selon l'une des revendications 4 ou 5, **caractérisée en ce que** la longueur de ladite au moins une ceinture de renvoi (34) est modifiable.

7. Orthèse dorsale (10) selon l'une des revendications 3, 5 ou 6, lorsqu'elle dépend de la revendication 5, **caractérisée en ce que** la pelote dorsale (30) est reliée à l'élément dorsal (12) de manière à pouvoir être déplacée, en particulier au moyen d'un mécanisme de déplacement ou d'une fermeture autoagrippante.

8. Orthèse dorsale (10) selon l'une des revendications précédentes, **caractérisée en ce que** l'élément de guidage (50) peut être monté ou fixé sur l'ensemble ceinture de hanche (20) et peut pivoter par rapport à l'ensemble ceinture de hanche (20).

9. Orthèse dorsale (10) selon l'une des revendications précédentes, **caractérisée en ce que** la longueur de l'ensemble ceinture de hanche (20) est réglable.

10. Orthèse dorsale (10) selon l'une des revendications précédentes, **caractérisée en ce que** l'ensemble ceinture de hanche (20) comprend une pelote ventrale (24) qui peut être accouplée ou est accouplée à l'ensemble ceinture d'épaule (40), la pelote ventrale (24) étant de préférence réalisée en plusieurs parties, de sorte qu'elle comprend au moins un premier et un deuxième élément de pelote ventrale (26, 28) pouvant être reliés entre eux et séparés l'un de l'autre, le premier et le deuxième élément de pelote ventrale (26, 28) étant de préférence reliés entre eux de manière amovible par une fermeture autoagrippante.

11. Orthèse dorsale (10) selon la revendication 10, **caractérisée en ce que** l'ensemble ceinture de hanche (20) comprend au moins une sangle de hanche (22) qui s'étend depuis l'élément dorsal (12) vers le pelote ventrale (24) et dont la longueur est réglable.

12. Orthèse dorsale (10) selon la revendication 10, **caractérisée en ce que** l'ensemble ceinture de hanche (20) comprend deux sangles de hanche (22), une sangle de hanche (22) et un des harnais d'épaule (42, 44) pouvant être montés ou fixés, respectivement, sur un élément de pelote ventrale (26, 28).

13. Orthèse dorsale (10) selon l'une des revendications précédentes, **caractérisée en ce que** le corps stabilisateur (14) s'étend sensiblement le long de la colonne vertébrale du patient.

14. Procédé de mise en place d'une orthèse dorsale (10) selon l'une des revendications 1 à 13, dans lequel les étapes suivantes sont mises en oeuvre :
- placer l'ensemble ceinture d'épaule (40) sur les épaules du patient ;
- entourer le tronc du patient avec l'ensemble ceinture de hanche (20) ; et
- régler la longueur d'au moins un des harnais d'épaule (42, 44).
